(19) Europäisches Patentamt
European Patent Office
Office européen des brevets

(11) Veröffentlichungsnummer: **0 393 500 A1**

# (12) EUROPÄISCHE PATENTANMELDUNG

(21) Anmeldenummer: **90106990.6**

(51) Int. Cl.5: **C07D 237/20, A61K 31/50**

(22) Anmeldetag: **11.04.90**

(30) Priorität: **17.04.89 CH 1424/89**

(43) Veröffentlichungstag der Anmeldung:
**24.10.90 Patentblatt 90/43**

(84) Benannte Vertragsstaaten:
**GR**

(71) Anmelder: **Byk Gulden Lomberg Chemische Fabrik GmbH**
**Byk-Gulden-Strasse 2**
**D-7750 Konstanz(DE)**

(72) Erfinder: **Amschler, Hermann, Dr.**
**Hohenhewenstrasse 19**
**D-7760 Radolfzell(DE)**
Erfinder: **Ulrich, Wolf-Rüdiger, Dr.**
**Hebelstrasse 3**
**D-7750 Konstanz(DE)**
Erfinder: **Beume, Rolf, Dr.**
**Bohlstrasse 13**
**D-7750 Konstanz 18(DE)**
Erfinder: **Eistetter, Klaus, Dr.**
**Säntisbick 7**
**D-7750 Konstanz 19(DE)**
Erfinder: **Eltze, Manfrid, Dr.**
**Schützenstrasse 20**
**D-7750 Konstanz(DE)**
Erfinder: **Flockerzi, Dieter, Dr.**
**Ackerweg 26**
**D-7753 Allensbach(DE)**
Erfinder: **Kilian, Ulrich, Dr.**
**Am Dachsberg 18**
**D-7752 Reichenau 2(DE)**
Erfinder: **Schudt, Christian, Dr.**
**Hoheneggstrasse 102**
**D-7750 Konstanz(DE)**

## (54) Neue Arylpyridazine, ihre Herstellung, Verwendung und sie enthaltende Arzneimittel.

(57) Verbindungen der allgemeinen Formel I

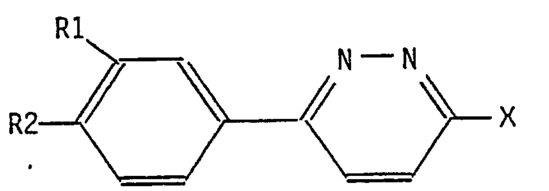

(I),

worin einer der Substituenten R1 und R2 Methoxy, Difluormethoxy oder Ethoxy und der andere C1-C5-Alkoxy, C4-C7-Cycloalkoxy, C3-C7-Cycloalkylmethoxy, C3-C5-Alkenyloxy oder C1-C4-Polyfluoralkoxy und X Cyanamino bedeutet, und ihre pharmakologisch verträglichen Salze mit Basen sind neu und eignen sich als bronchospasmolytische Wirkstoffe.

EP 0 393 500 A1

Xerox Copy Centre

# Neue Arylpyridazine, ihre Herstellung, Verwendung und sie enthaltende Arzneimittel

## Technisches Gebiet

Die Erfindung betrifft 6-Aryl-3-cyanaminopyridazine, ihre Herstellung, Verwendung und sie enthaltende Arzneimittel.

## Stand der Technik

6-Aryl-3-cyanaminopyridazine sind nicht bekannt.

## Beschreibung der Erfindung

Es wurde gefunden, daß bestimmte 6-Aryl-3-cyanaminopyridazine vorteilhafte pharmakologische Wirkungen aufweisen.

Gegenstand der Erfindung sind 6-Aryl-3-cyanaminopyridazine der allgemeinen Formel I

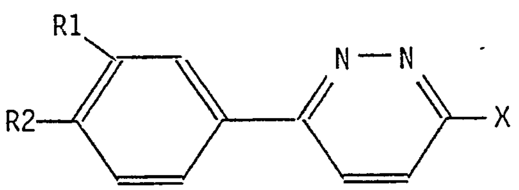 (I),

worin einer der Substituenten R1 und R2 Methoxy, Difluormethoxy oder Ethoxy, und der andere C1-C5-Alkoxy, C4-C7-Cycloalkoxy, C3-C7-Cycloalkylmethoxy, C3-C5-Alkenyloxy oder C1-C4-Polyfluoralkoxy bedeutet, X Cyanamino bedeutet, und ihre Salze mit Basen.

C1-C5-Alkoxy ist geradkettig oder verzweigt. Als beispielhafte C1-C5-Alkoxyreste seien genannt der Methoxy-, Ethoxy-, n-Propoxy-, Isopropoxy-, n-Butoxy-, Isobutoxy-, sec.-Butoxy-, tert-Butoxy-, n-Pentyloxy, Isopentyloxy- und der 2,2-Dimethylpropoxyrest. C3-C4-Alkoxy ist bevorzugt.

C4-C7-Cycloalkoxy steht beispielsweise für Cyclobutyloxy, Cyclopentyloxy, Cyclohexyloxy und Cycloheptyloxy, wovon Cyclopentyloxy bevorzugt ist.

C3-C7-Cycloalkylmethoxy steht beispielsweise für Cyclopropylmethoxy, Cyclobutylmethoxy, Cyclopentylmethoxy, Cyclohexylmethoxy und Cycloheptylmethoxy, wovon Cyclopropylmethoxy und Cyclobutylmethoxy bevorzugt sind.

C3-C5-Alkenyloxy ist geradkettig oder verzweigt. Die Doppelbindung von Alkenyloxy geht nicht von dem Kohlenstoffatom aus, das an das Sauerstoffatom bindet. Als beispielhafte C3-C5-Alkenyloxyreste seien genannt der Buten-2-yloxy-, der Allyloxy- und der Methallyloxyrest.

C1-C5-Alkoxy ist gegenüber C3-C5-Alkenyloxy bevorzugt.

Unter C1-C4-Polyfluoralkoxy wird geradkettiges oder verzweigtes C1-C4-Alkoxy verstanden, bei dem mindestens 2 Wasserstoffatome durch Fluor ersetzt sind. Geradkettiges C1-C3-Alkoxy, bei dem mindestens 2 Wasserstoffatome durch Fluor ersetzt sind, ist bevorzugt. Bevorzugte C1-C4-Polyfluoralkoxygruppen sind Trifluormethoxy, 1,1,2,2-Tetrafluorethoxy und insbesondere Difluormethoxy und 2.2.2-Trifluorethoxy.

Unter Cyanamino wird die Gruppe -NHCN verstanden.

Als Salze kommen Salze mit anorganischen und organischen Basen in Betracht. Besonders erwähnt seien die pharmakologisch verträglichen Salze der in der Galenik üblicherweise verwendeten anorganisch und organischen Basen. Pharmakologisch unverträgliche Salze, die beispielsweise bei der Herstellung der erfindungsgemäß Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Salze mit Basen, wobei als Kationen für die Salzbildung vor allem die Kationen der Alkalimetalle oder Erdalkalimetalle verwendet werden; es kommen jedoch auch die entsprechenden Kationen organischer Stickstoffbasen, wie Amine oder Aminoalkanole, Aminozucker etc. zur Anwendung. Beispielsweise seien die Salze von Natrium, Magnesium,

Calcium, Dimethylamin, Diethylamin, Ethanolamin, Diethanolamin, Triethanolamin, Glucamin, N-Methylglucamin (Meglumin), Glucosamin, N-Methylglucosamin genannt.

Eine Ausgestaltung (Ausgestaltung a) der Erfindung sind 6-Aryl-3-cyanaminopyridazine der oben angegebenen allgemeinen Formel I, worin

R1 Methoxy, Difluormethoxy oder Ethoxy,

R2 C1-C4-Alkoxy, C4-C6-Cycloalkoxy, C3-C6-Cycloalkylmethoxy, C3-C4-Alkenyloxy oder C1-C2-Polyfluoralkoxy und

X Cyanamino bedeuten,

und ihre Salze mit Basen.

Eine weitere Ausgestaltung (Ausgestaltung b) der Erfindung sind 6-Aryl-3-cyanaminopyridazine der oben angegebenen allgemeinen Formel I, worin

R1 C2-C4-Alkoxy, C4-C6-Cycloalkoxy, C3-C6-Cycloalkylmethoxy, C3-C4-Alkenyloxy oder C1-C2-Polyfluoralkoxy,

R2 Methoxy, Difluormethoxy oder Ethoxy und

X Cyanamino bedeuten,

und ihre Salze mit Basen.

Bevorzugte erfindungsgemäße Verbindungen sind solche der Formel I, worin einer der Substituenten R1 und R2 Methoxy, Difluormethoxy oder Ethoxy, und der andere C1-C4-Alkoxy, C4-C6-Cycloalkoxy, C3-C6-Cycloalkylmethoxy oder C1-C2-Polyfluoralkoxy bedeutet und X Cyanamino bedeutet, und ihre Salze mit Basen.

Bevorzugte Vertreter der Ausgestaltung a sind solche, in denen R2 C1-C4-Alkoxy, C3-C6-Cycloalkylmethoxy oder C1-C2-Polyfluoralkoxy bedeutet.

Bevorzugte Vertreter der Ausgestaltung b sind solche, in denen R1 C2-C4-Alkoxy, C4-C6-Cycloalkoxy, C3-C6-Cycloalkylmethoxy oder C1-C2-Polyfluoralkoxy bedeutet.

Die Ausgestaltung b ist gegenüber der Ausgestaltung a bevorzugt.

Besonders bevorzugte erfindungsgemäße Verbindungen sind solche der Formel I, worin R1 C2-C4-Alkoxy, Cyclopentyloxy, Cyclopropylmethoxy, Cyclobutylmethoxy, Difluormethoxy oder 2,2,2-Trifluorethoxy bedeutet, R2 Methoxy, Ethoxy oder Difluormethoxy bedeutet und X Cyanamino bedeutet, und ihre pharmakologisch verträglichen Salze mit Basen.

Die erfindungsgemäßen 6-Aryl-3-cyanaminopyridazine können als tautomere Formen vorliegen. Das Proton der 3-Cyanaminogruppe vermag zwischen dieser Gruppe und dem Stickstoff in 2-Stellung des Pyridazinrings zu wandern. Erfindungsgemäß ist bei Angabe oder Darstellung nur eines Tautomeren jeweils auch das andere Tautomere zu verstehen.

Ein weiterer Gegenstand der Erfindung ist die Verwendung der erfindungsgemäßen Verbindungen bei der Behandlung oder Prophylaxe von Krankheiten, die auf der Erkrankung der Bronchien beruhen.

Ein weiterer Gegenstand der Erfindung ist die Verwendung der erfindungsgemäßen Verbindungen und ihrer pharmakologisch verträglichen Salze zur Herstellung von Arzneimitteln zur Behandlung und/oder Prophylaxe von Erkrankungen der Bronchien.

Gegenstand der Erfindung ist ferner ein Verfahren zur Herstellung der 3-Cyanamino-6-arylpyridazine der allgemeinen Formel I, worin R1, R2 und X die oben angegebenen Bedeutungen haben, und ihrer Salze mit Basen, das dadurch gekennzeichnet ist, daß man eine Verbindung der allgemeinen Formel I, worin R1 und R2 die oben angegebene Bedeutung besitzen und X eine nucleophil verdrängbare Abgangsgruppe bedeutet, mit Alkalicyanamid, bevorzugt Natriumcyanamid in Gegenwart eines Phasentransferkatalysators in einem wasserfreien, inerten Lösungsmittel umsetzt.

Unter eine nucleophil verdrängbaren Abgangsgruppe wird insbesondere ein Halogenatom verstanden, wobei Chlor und Brom besonders in Frage kommen.

Die Technik der Phasentransferkatalyse ist dem Fachmann bekannt (siehe z.B. Jozef Dockx, Synthesis 1973, 441-456). Als Phasentransferkatalysatoren kommen die für nucleophile Verdrängungen, insbesondere für Halogenaustausch gebräuchlichen in Frage. Geeignet sind z.B. Kronenether, quartäre Phosphoniumsalze und insbesondere quartäre Ammoniumsalze, wie z.B. Tetrabutylammoniumchlorid.

Beispielsweise werden die Ausgangsverbindungen in einem Kohlenwasserstoff, wie z.B. Toluol oder Xylol, oder in einem Ether, wie z.B. Dioxan, oder einem Keton, wie z.B. 2-Methyl-4-pentanon (Isobutylmethylketon), oder einem N,N-disubstituierten Säureamid, wie Z.B. Dimethylformamid oder N-Methylpyrrolidon unter wasserfreien Bedingungen in Gegenwart von 0,1 bis 2 Mol des Phasentransferkatalysators bei Temperaturen von 50 bis 200°C, insbesondere von 80 bis 150°C, bevorzugt beim Siedepunkt des Lösungsmittels, mit mindestens 2 Mol Alkalicyanamid umgesetzt.

Die Überführung der 6-Aryl-3-cyanaminopyridazine in die Salze erfolgt nach dem Fachmann bekannten Methoden. Als alkalischer Reaktionspartner wird diejenige anorganische oder organische Base verwendet,

deren Salz gewünscht wird. Man erhält die Salze beispielsweise, indem man die 6-Aryl-3-cyanaminopyridazine mit dem stöchiometrischen Äquivalent an entsprechender Base, z.B. Natriumhydroxid oder Natriummethanolat umsetzt, oder leicht lösliche Salze durch doppelte Umsetzung in schwer lösliche Salze überführt.

Für die Herstellung der Verbindungen der Ausgestaltungen a und b werden entsprechende Ausgangsverbindungen der allgemeinen Formel I, worin R1 und R2 die oben jeweils angegebene Bedeutung haben, und X eine nucleophil verdrängbare Abgangsgruppe bedeutet, eingesetzt.

Die Ausgangsverbindungen der allgemeinen Formel I, worin X eine nucleophil verdrängbare Abgangs gruppe bedeutet, sind neu und ebenfalls Gegenstands der Erfindung. Sie können nach bekannten Verfahren hergestellt werden beispielsweise durch Umsetzung von 6-Aryl-3[2H]pyridazinonen der Formel II,

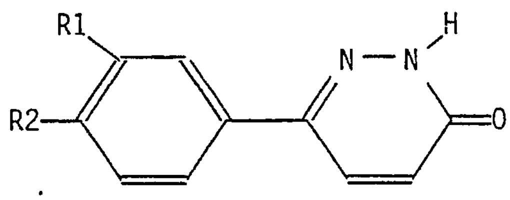

(II),

worin R1 und R2 die oben jeweils angegebene Bedeutung haben, in einem inerten Lösungsmittel, wie Toluol der Xylol, oder ohne Lösungsmittel, mit Phosphoroxitrihalogenid, wie Phosphoroxitrichlorid, im Überschuß.

Die Ausgangsverbindungen der Formel II sind bekannt oder sie können nach bekannten Verfahren hergestellt werden, wie sie z.B. im Europäischen Patent 163 965 beschrieben sind.

Die folgenden Beispiele dienen zur näheren Erläuterung der Erfindung. Fp. bedeutet Schmelzpunkt, Sdp. bedeutet Siedepunkt.

## Beispiele

### Endprodukte

**1.** 3-Cyanamino-6-(3-methoxy-4-n-propoxyphenyl)pyridazin

1,8 g 3-Chlor-6-(3-methoxy-4-n-propoxyphenyl)pyridazin, 0,8 g Natriumcyanamid und 3,6 g Tetrabutylammoniumchlorid werden in 30 ml trockenem Toluol 5 Stunden am Rückfluß gekocht. Danach wird das Reaktionsgemisch im Vakuum eingedampft und der Rückstand mit 200 ml 2N Natriumhydrogensulfat-Lösung unter gutem Rühren kurz aufgekocht, wobei das Reaktionsprodukt als feiner kristalliner Niederschlag ausfällt. Er wird abgesaugt, mit Wasser gewaschen und im Vakuum getrocknet. Zur Reinigung wird er mit 50 ml Isopropanol kurz aufgekocht, abgesaugt und im Vakuum getrocknet. Man erhält 1,5 g (83,3 %) der Titelverbindung vom Fp. 187°C.

**2.** 3-Cyanamino-6-(3-ethoxy-4-methoxyphenyl)pyridazin

2,7 g 3-Chlor-6-(3-ethoxy-4-methoxyphenyl)pyridazin, 1,3 g Natriumcyanamid und 0,5 g Benzyltriethylammoniumchlorid werden in 30 ml N-Methylpyrrolidon 3 Stunden auf 150°C erhitzt. Nach dem Abkühlen wird die Reaktionslösung mit 200 ml 1N Schwefelsäure verdünnt und 3 mal mit je 50 ml Chloroform extrahiert. Die vereinigten Extrakte werden über Natriumsulfat getrocknet und eingedampft, der Rückstand wird in Isopropanol aufgekocht. Das kristalline Produkt wird abgesaugt, mit Isopropanol ausgewaschen und im Vakuum getrocknet. Man erhält 2,6 g (96,3 %) der Titelverbindung vom Fp. 206°C.

**3.** 3-Cyanamino-6-[4-(2-methylpropoxy)-3-methoxyphenyl]pyridazin

3 g 3-Brom-6-[4-(2-methylpropoxy)3-methoxyphenyl]pyridazin, 1,3 g Natriumcyanamid und 0,5 g 18-Krone-6 werden in 30 ml 2-Methyl-4-pentanon 5 Stunden am Rückfluß gekocht. Die Reaktionslösung wird

anschließend im Vakuum eingedampft und der Rückstand mit 200 ml 2N Natriumhydrogensulfat-Lösung unter gutem Rühren kurz aufgekocht, wobei das Reaktionsprodukt als feiner, kristalliner Niederschlag ausfällt. Er wird abgesaugt, mit Isopropanol nochmals aufgekocht, gekühlt, abgesaugt und nach Waschen mit Isopropanol und Petroleumbenzin (Sdp. 50 - 70°C) im Vakuum getrocknet. Man erhält 2,5 g (80,6 %) der Titelverbindung vom Fp. 191°C.

4. 3-Cyanamino-6-[4-(3-methylbut-1-oxy)-3-methoxyphenyl]pyridazin

6 g 3-Chlor-6-[4-(3-methylbut-1-oxy)-3-methoxyphenyl]pyridazin, 2,5 g Natriumcyanamid und 10,3 g Tetrabutylammoniumchlorid werden in 80 ml Dioxan 6 Stunden am Rückfluß gekocht. Die Reaktionsmischung wird nach dem Abkühlen mit 200 ml 2N Schwefelsäure verdünnt, wobei sich das Reaktionsprodukt kristallin abscheidet. Der Niederschlag wird abgesaugt, mit Wasser neutral gewaschen, nochmals mit Aceton aufgeschlämmt, abgesaugt und im Vakuum getrocknet. Man erhält 3,6 g (59 %) der Titelverbindung vom Fp. 223°C.

Analog erhält man unter Einsatz entsprechender 3-Chlor-6-arylpyridazine:
3-Cyanamino-6-[4-methoxy-3-(1-methylethoxy)phenyl]pyridazin, Fp. 210°C (89 %),
3-Cyanamino-6-[4-methoxy-3-(2-methylpropoxy)phenyl]pyridazin, Fp. 213°C (70 %),
3-Cyanamino-6-[3-methoxy-4-(1-methylethoxy)phenyl]pyridazin, Fp. 223°C (59 %),
3-Cyanamino-6-(4-ethoxy-3-methoxyphenyl)pyridazin, Fp. 221°C (56 %),
3-Cyanamino-6-(4-difluormethoxy-3-methoxyphenyl)pyridazin, Fp. 209°C (83 %),
3-Cyanamino-6-(3-difluormethoxy-4-methoxyphenyl)pyridazin, Fp. 226°C (79 %),
3-Cyanamino-6-(3,4-dimethoxy-phenyl)pyridazin, Fp. 246°C (78 %),
3-Cyanamino-6-(3-cyclopentyloxy-4-methoxyphenyl)pyridazin, Fp. 231°C (92 %),
3-Cyanamino-6-(4-difluormethoxy-3-ethoxyphenyl)pyridazin, Fp. 188°C (74 %),
3-Cyanamino-6-[4-ethoxy-3-(2-methylpropoxy)phenyl]pyridazin, Fp. 203°C (82 %),
3-Cyanamino-6-(3-difluormethoxy-4-ethoxyphenyl)pyridazin, Fp. 212°C (74 %),
3-Cyanamino-6-(3-cyclobutylmethoxy-4-methoxyphenyl)pyridazin, Fp. 202°C (95 %),
3-Cyanamino-6-[4-difluormethoxy-3-(1-methylethoxy)phenyl]pyridazin, Fp. 188°C (94 %),
3-Cyanamino-6-(3-cyclopentyloxy-4-difluormethoxyphenyl)pyridazin, Fp. 215°C (90 %),
Fp. des Megluminsalzes: 124°C,
3-Cyanamino-6-(3-cyclopropylmethoxy-4-difluormethoxyphenyl)pyridazin, Fp. 189°C (96 %),
3-Cyanamino-6-[4-difluormethoxy-3-(2-methylpropoxy)phenyl]pyridazin, Fp. 203-204°C (45 %),
3-Cyanamino-6-[4-difluormethoxy-3-(2,2,2-trifluorethoxy)phenyl]pyridazin, Fp. 200°C (98 %),
Fp. des Natriumsalzes: 290°C,
3-Cyanamino-6-(4-cyclopropylmethoxy-3-difluormethoxyphenyl)pyridazin, Fp. 194°C (95 %).

Ausgangsverbindungen

3-Chlor-6-(3-methoxy-4-n-propoxyphenyl)pyridazin

18,0 g 6-(3-Methoxy-4-n-propoxyphenyl)-3[2H]pyridazinon werden in 63,6 g Phosphoroxitrichlorid suspendiert. Die Mischung wird bei 100°C so lange gerührt, bis eine klare Lösung entstanden ist und die HCl-Entwicklung aufhört. Man gießt die auf ca. 50°C abgekühlte Lösung unter ständigem Rühren auf 1 kg Eis, wobei sich sofort ein kristalliner Niederschlag bildet, der abgesaugt und mit Wasser, verdünnter Natriumbicarbonat-Lösung und nochmals mit Wasser säurefrei gewaschen wird. Nach dem Trocknen im Vakuum erhält man 19,1 g (98,8 %) der Titelverbindung vom Fp. 129°C nach Kristallisieren aus Cyclohexan vom Fp. 132°C.

Analog erhält man unter Einsatz entsprechender 6-Aryl-3[2H]pyridazinone folgende 3-Chlor-6-arylpyridazine:
3-Chlor-6-[4-methoxy-3-(1-methylethoxy)phenyl]pyridazin, Fp. 148°C (92 %),
3-Chlor-6-(3-ethoxy-4-methoxyphenyl)pyridazin, Fp. 141°C (63 %),
3-Chlor-6-[4-methoxy-3-(2-methylpropoxy)phenyl]pyridazin, Fp. 138°C (97 %),
3-Chlor-6-[3-methoxy-4-(2-methylpropoxy)phenyl]pyridazin, Fp. 116°C (95 %),
3-Chlor-6-(3-difluormethoxy-4-methoxyphenyl)pyridazin, Fp. 156°C (98 %),
3-Chlor-6-(4-difluormethoxy-3-methoxyphenyl)pyridazin, Fp. 155°C (97 %),

3-Chlor-6-[3-methoxy-4-(3-methylbutoxy)phenyl]pyridazin, Fp. 98°C (87 %),
3-Chlor-6-[3-methoxy-4-(1-methylethoxy)phenyl]pyridazin, Fp. 115°C (63 %),
3-Chlor-6-(4-ethoxy-3-methoxyphenyl)pyridazin, Fp. 145°C (94 %),
3-Chlor-6-(3,4-dimethoxy-phenyl)pyridazin, Fp. 158°C (76 %),
3-Chlor-6-(3-cyclopentyloxy-4-methoxyphenyl)pyridazin, Fp. 141°C (80 %),
3-Chlor-6-[4-ethoxy-3-(2-methylpropoxy)phenyl]pyridazin, Fp. 155°C (77 %),
3-Chlor-6-(4-difluormethoxy-3-ethoxyphenyl)pyridazin, Fp. 125°C (87 %),
3-Chlor-6-(3-difluormethoxy-4-ethoxyphenyl)pyridazin, Fp. 165°C (94 %),
3-Chlor-6-(3-cyclobutylmethoxy-4-methoxyphenyl)pyridazin, Fp. 159°C (98 %),
3-Chlor-6-(3-cyclopentyloxy-4-difluormethoxyphenyl)pyridazin, Fp. 95,5-96°C(68 %),
3-Chlor-6-[4-difluormethoxy-3-(1-methylethoxy)phenyl]pyridazin, Fp. 90°C (97 %),
3-Chlor-6-(3-cyclopropylmethoxy-4-difluormethoxyphenyl)pyridazin, Fp. 140°C (35 %),
3-Chlor-6-[4-difluormethoxy-3-(2,2,2-trifluorethoxy)phenyl]pyridazin, Fp. 109°C (93 %),
3-Chlor-6-(4-cyclopropylmethoxy-3-difluormethoxyphenyl)pyridazin, Fp. 122,5°C (12 %),
3-Chlor-6-[4-difluormethoxy-3-(2-methylpropoxy)phenyl]pyridazin, Fp. 121-122°C (97 %).

Gewerbliche Anwendbarkeit

Die erfindungsgemäßen 6-Aryl-3-cyanaminopyridazine besitzen wertvolle pharmakologische Eigenschaften, die sie gewerblich verwertbar machen. Sie zeichnen sich vor allem durch solche Eigenschaften aus, die sie für die Therapie von Atemwegserkrankungen verschiedener Genese geeignet erscheinen lassen. Insbesondere können entzündliche und allergeninduzierte Bronchialerkrankungen aufgrund der antiinflammatorischen und broncholytischen Wirksamkeit der erfindungsgemäßen Verbindungen behandelt werden. Daneben zeichnen sich die erfindungsgemäßen Verbindungen durch eine geringe Toxizität, eine große therapeutische Breite und das Fehlen wesentlicher Nebenwirkungen aus.

Die broncholytische und antiinflammatorische Wirksamkeit der 6-Aryl-3-cyanaminopyridazine ermöglicht ihren Einsatz in der Human- und Veterinärmedizin, wobei sie zur Behandlung und Prophylaxe von Krankheiten, die auf Erkrankungen der Bronchien beruhen, verwendet werden. Beispielsweise können akute und chronisch obstruktive Atemwegserkrankungen verschiedener Genese (Bronchitis, allergische Bronchitis, Asthma bronchiale) bei Mensch und Tier behandelt werden.

Ein weiterer Gegenstand der Erfindung ist daher ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe von Krankheiten, die auf Erkrankungen der Bronchien beruhen.

Ebenso betrifft die Erfindung die Verwendung der erfindungsgemäßen Verbindungen zur Herstellung von Arzneimitteln, die zur Behandlung und/oder Prophylaxe von Krankheiten, die auf Erkrankungen der Bronchien beruhen, eingesetzt werden.

Weiterhin sind Arzneimittel zur Behandlung und/oder Prophylaxe von Krankheiten, die auf Erkrankungen der Bronchien beruhen und die eine oder mehrere der erfindungsgemäßen Verbindungen und/oder ihre pharmakologisch verträglichen Salze enthalten, Gegenstand der Erfindung.

Die erfindungsgemäßen Arzneimittel werden nach an sich bekannten Verfahren hergestellt, wobei bezüglich der Zubereitungen, Dosierungen, Darreichungsformen etc. beispielsweise auf die Ausführungen im Europäischen Patent 163 965 verwiesen wird.

Beispiele für Arzneimittelzubereitungen

Tabletten mit 100 mg 3-Cyanamino-6-[4-(2-methylpropoxy)-3-methoxyphenyl]pyridazin

40 kg Wirkstoff, 24 kg Milchzucker und 16 kg Maisstärke werden mit 4 kg Polyvinylpyrrolidon (MG ca. 25000) in 5,5 Liter Wasser granuliert und durch ein Sieb von 1,25 mm Maschenweite gepreßt. Nach dem Trocknen werden 10 kg Carboxymethylcellulose, 4 kg Talkum und 2 kg Magnesiumstearat zugegeben. Auf einer Exzentermaschine wird das Granulat zu Tabletten von 9 mm Durchmesser, 250 mg Gewicht und einer Härte von 4 bis 5 kg verpreßt.

Kapseln mit 15 mg 3-Cyanamino-6-[3-(1-methylethoxy)-4-methoxyphenyl]pyridazin

150 mg Wirkstoff, 845 mg mikrokristalline Cellulose und 5 mg amorphe Kieselsäure werden feingepulvert, gut vermischt und in Hartgelatinekapseln Größe 4 abgefüllt.

Dosieraerosolzubereitung enthaltend 3-Cyanamino-6-(3-ethoxy-4-methoxyphenyl)pyridazin

0,540 g Span®85 und 0,135 g Aroma werden in 10,215 g gekühltem Frigen®11 gelöst. In die Lösung werden 0,270 g mikronisierter Wirkstoff eingerührt und in 24 ml-Dosen eingefüllt. Nach dem Vercrimpen werden 14,971 g Frigen®12 eingepreßt. Bei einem Kammervolumen des Dosierventils von 125 µl werden pro Ventilhub 1,6 mg Wirkstoff als Aerosol freigesetzt.

Biologische Untersuchungen

In den nachfolgenden Tabellen sind die untersuchten Verbindungen durch Nummern gekennzeichnet:

1 3-Cyanamino-6-(3-methoxy-4-n-propoxyphenyl)pyridazin
2 3-Cyanamino-6-(3-ethoxy-4-methoxyphenyl)pyridazin
3 3-Cyanamino-6-[4-methoxy-3-(1-methylethoxy)phenyl]pyridazin
4 3-Cyanamino-6-[4-methoxy-3-(2-methylpropoxy)phenyl]pyridazin
5 3-Cyanamino-6-[3-methoxy-4-(2-methylpropoxy)phenyl]pyridazin
6 3-Cyanamino-6-[3-methoxy-4-(1-methylethoxy)phenyl]pyridazin
7 3-Cyanamino-6-(4-ethoxy-3-methoxyphenyl)pyridazin
8 3-Cyanamino-6-(4-difluormethoxy-3-methoxyphenyl)pyridazin
9 3-Cyanamino-6-(3-difluormethoxy-4-methoxyphenyl)pyridazin
10 3-Cyanamino-6-(3,4-dimethoxyphenyl)pyridazin
11 3-Cyanamino-6-(3-cyclopentyloxy-4-methoxyphenyl)pyridazin
12 3-Cyanamino-6-(4-difluormethoxy-3-ethoxyphenyl)pyridazin
13 3-Cyanamino-6-[4-ethoxy-3-(2-methylpropoxy)phenyl]pyridazin
14 3-Cyanamino-6-(3-difluormethoxy-4-ethoxyphenyl)pyridazin
15 3-Cyanamino-6-(3-cyclobutylmethoxy-4-methoxyphenyl)pyridazin
16 3-Cyanamino-6-[4-difluormethoxy-3-(1-methylethoxy)phenyl]pyridazin
17 3-Cyanamino-6-(3-cyclopentyloxy-4-difluormethoxyphenyl)pyridazin
18 3-Cyanamino-6-(3-cyclopropylmethoxy-4-difluormethoxyphenyl)pyridazin
19 3-Cyanamino-6-[4-difluormethoxy-3-(2-methylpropoxy)phenyl]pyridazin
20 3-Cyanamino-6-[4-difluormethoxy-3-(2,2,-trifluorethoxy)phenyl]pyridazin
21 3-Cyanamino-6-(4-cyclopropylmethoxy-3-difluormethoxyphenyl)pyridazin

Die bronchospasmolytische Wirkung der Verbindungen auf die Trachealspangen-Kette des Meerschweinchens wurde in vitro wie folgt geprüft:
Vier parallele, aus jeweils 6 Einzelringen bestehende Trachealspangen-Ketten des Meerschweinchens (weibl. und männl., 430 - 600 g) im Organbad [5 ml, Krebs-Henseleit-Lösung mit Zusatz von Phentolamin ($10^{-5}$ mol/l), 37°C, Vorspannung der Organe 2 g, Begasung mit Carbogen] entwickeln nach etwa 20 bis 30 Minuten eine stabile, tonische Spontankontraktur. An diesen dauerkontrahierten Organen kann unter isometrischen Meßbedingungen durch Applikation der Prüfsubstanz in kumulativ-halblogarithmisch ansteigender Konzentration (z.B. $1x10^{-6}$ + $2x10^{-6}$ + $7x10^{-6}$ + $2x10^{-5}$ usw. mol/l) eine Relaxation herbeigeführt werden, wobei nach jeder Einzeldosis der Testsubstanz eine konstante Relaxations-Antwort abgewartet wird, bevor die nächst höhere Konzentration appliziert wird. Über einen Zeitraum von 20 bis 30 Minuten wird somit eine vollständige Dosis-Wirkungskurve der Testsubstanz erhalten. Die jeweilige Relaxation wird als Prozentbruchteil der durch Gabe von (-)Isoprenalin ($10^{-6}$ mol/l) maximal erreichbaren Relaxation ausgedrückt. Als Maß für die bronchodilatorische Aktivität dient die Konzentration der Testsubstanz, welche 50 % der maximal erreichbaren Relaxation bewirkt, ausgedrückt durch den negativen Logarithmus der $EC_{50}$ mol/l: -1g[$EC_{50}$].

In der Tabelle 1 sind die Werte -1g[$EC_{50}$] und die Quotienten aus den $EC_{50}$-Werten für Theophyllin und die untersuchte Substanz angegeben. Die gefundenen Werte zeigen eine große Überlegenheit der erfindungsgemäßen Verbindungen gegenüber Theophyllin bezüglich der bronchospasmolytischen Aktivität.

Tabelle 1:

| Lfd. Nr. | $-lg[EC_{50}]$ | $[EC_{50}]_{Theophyllin}$ / $[EC_{50}]_{Substanz}$ |
|---|---|---|
| 1 | 5,25 | 24,0 |
| 2 | 5,52 | 44,7 |
| 3 | 6,38 | 324 |
| 4 | 6,95 | 1203 |
| 5 | 5,45 | 38,1 |
| 6 | 5,09 | 16,6 |
| 7 | 5,19 | 20,9 |
| 8 | 5,73 | 72,5 |
| 9 | 5,63 | 57,6 |
| 10 | 5,15 | 19,1 |
| 11 | 6,25 | 240 |
| 12 | 5,61 | 55,0 |
| 13 | 6,27 | 251 |
| 14 | 5,69 | 66,1 |
| 15 | 6,04 | 148 |
| 16 | 6,18 | 204 |
| 17 | 6,12 | 178 |
| 18 | 6,25 | 240 |
| 19 | 5,85 | 95,6 |
| 20 | 5,64 | 58,9 |
| 21 | 6,00 | 135 |
| Theophyllin | 3,87 | 1 |

Für die Verbindungen 3 und 4 wurde die trachearelaxierende Wirkung zusätzlich durch Relaxierung der durch Histamin (His), Carbachol (CC), Prostaglandin ($PGF_{2alpha}$), Ovalbumin (OA) und Leukotrien ($LTC_4$) erzeugten Kontrakturen geprüft. Wie aus den in Tabelle 2 angegebenen Meßwerten zu entnehmen ist, zeigen die erfindungsgemäßen Verbindungen auch unter den modifizierten Bedingungen eine gegenüber Theophyllin stark erhöhte Relaxation.

Tabelle 2

| Kontraktion durch | 3 | 4 | Theophyllin |
|---|---|---|---|
| His ($10^{-5}$) | 6,15 | 6,64 | 3,76 |
| CC ($10^{-6}$ M) | 5,59 | 5,98 | 3,24 |
| $PGF_{2alpha}$ ($10^{-6}$ M) | 6,35 | 6,82 | 3,77 |
| OA ($10^{-5}$ - $10^{-4}$ mg/ml) | 6,19 | 6,40 | 3,54 |
| $LTC_4$ (5 x $10^{-8}$ M) | 6,19 | 6,84 | 3,67 |

Die bronchospasmolytische Wirkung wurde weiterhin am Modell "Histamininduzierter Bronchospasmus am narkotisierten Meerschweinchen" bestimmt:

Bei diesem Modell werden pharmakodynamische bzw. toxische Effekte an inneren sensiblen Rezeptoren, auf die Atmung und am Herz-Kreislauf-System vom Meerschweinchen simultan registriet [U. Kilian, E. Müller, E. Ch. Dittmann und J. Hamacher, Arzneimittel-Forschung 28 (II) 1699-1708, 1978]. An narkotisierten (Ethylurethan 1,25 g/kg i.p.) monovagotomierten, spontanatmenden Meerschweinchen (männl., 350 - 450 g) wurde das Pneumotachogramm registriert und zur Charakterisierung des durch Histamin (0,09 -0,18 mol/kg i.v.) ausgelösten Bronchospasmus die maximale Strömungsgeschwindigkeit der Atemluft während der Expiration ($Vmax_e$) gemessen.

Ein Histaminspasmus vor Substanzgabe wurde mit mehreren Histaminspasmen auch Substanzgabe verglichen. Die Prüfsubstanzen wurden intravenös (i.v.) und/oder intrajejunal (i.j.) appliziert.

Es wurde gefunden, daß die untersuchten Verbindungen den Histamin-induzierten Bronchospasmus am narkotisierten Meerschweinchen etwa 2 - 5 mal stärker hemmen als Theophyllin.

Tabelle 3:

| Mittlere prozentuale bronchospasmolytische Wirkung 0-1 Std. p. appl., gemessen an der Hemmung der histamininduzierten Abnahme von $Vmax_e$ | | | |
|---|---|---|---|
| Lfd. Nr. | Dosis µmol/kg | % Hemmung nach | |
| | | i.V.- | i.j.-Gabe |
| 3 | 20 | 28 | 29 |
| | 60 | 65 | 65 |
| 4 | 20 | 37 | 39 |
| | 60 | 87 | 60 |
| 12 | 20 | 50 | - |
| 13 | 20 | 28 | - |
| Theophyllin | 20 | 13 | 11 |
| | 60 | 25 | 37 |
| | 100 | 34 | 45 |

Zusätzlich wurde die bronchospasmolytische Wirkung am Modell "Schutzwirkung gegen den Acetylcholin-induzierten Bronchospasmus am wachen Meerschweinchen" geprüft:

Die Versuchsdurchführung erfolgt in Anlehnung an T. Olsson, Acta Allergologica 26, 438-447 (1971). Meerschweinchen (250 - 350 g) werden in einem verschlossenen Plexiglaszylinder (Volumen 5 l) vor Substanzgabe zweimal im Abstand von 20 Minuten sowie 30 Minuten nach Substanzgabe einem Acetylcholin-Nebel (0,06 % in 0,9 % Natriumchloridlösung; Ultraschallvernebler Heyer Use 77) ausgesetzt. Die Zeit vom Beginn der Verneblung bis zum Einsetzen deutlicher Atemanstrengungen (unter Umständen hypoxischer Krampfanfall in Seitenlage) wird gemessen und als Latenzzeit bezeichnet. Eine Verlängerung der Latenzzeit nach Substanzgabe auf mindestens die dreifache mittlere Latenzzeit vor Substanzgabe wird als Schutzwirkung angesehen und die Anzahl der in dem Kollektiv geschützten Tiere angegeben. Die Applikation der Prüfsubstanzen erfolgt oral mittels Schlundsonde (Dosis 100 µmol/kg, Volumen 1 ml/kg, Suspensionsmittel 4 %-ige Methocelsuspension in 0,9 %-iger Natriumchloridlösung).

Im Kontrollversuch (ohne Substanzapplikation) liegt die Latenzzeit bei 2 Minuten. Die Applikation der Prüfsubstanz erfolgt per oral mittels Schlundsonde (Standarddosis 100 µmol, Volumen 1 ml 4 %ige Methocelsuspension in 0,9 %iger Natriumchloridlösung/kg). Nach 30 Minuten werden die Tiere erneut dem Acetylcholin-Nebel ausgesetzt und die Latenzzeiten gemessen. Eine Verlängerung der Latenzzeit auf mindestens die dreifache Länge wird als Schutzwirkung angesehen.

Aus Tabelle 4 ist zu entnehmen, daß die untersuchten Verbindungen eine dem Theophyllin vergleichbare oder überlegene Schutzwirkung aufweisen.

Tabelle 4:

| Schutzwirkung gegen den Acetylcholin-induzierten Bronchospasmuns am wachen Meerschweinchen, ermittelt 30 Minuten nach oraler Substanz- bzw. Placebogabe. Dosis: 100 µmol/kg der Testsubstanz (= Verum) bzw. substanzfreies Suspensionsmittel (= lacebo). | | |
|---|---|---|
| Lfd. Nr. | Anzahl geschützter Tiere / Anzahl verwendeter Tiere | |
| | Verum | Placebo |
| 3 | 14/20 | 5/20 |
| 4 | 17/20 | 5/20 |
| 12 | 14/20 | 4/20 |
| 13 | 13/20 | 5/20 |
| 16 | 10/20 | 4/20 |
| 18 | 0/20 | 4/20 |
| 20 | 11/20 | 4/20 |
| Theophyllin | 6/20 | 2/20 |

Als besonders aussagekräftig für eine zu erwartende bronchospasmolytische und/oder antiinflammatorische Wirkung wird die Hemmung der Phosphodiesterasen der Klassen III (PDE III = hoch affine cAMP-PDE durch cGMP hemmbar) und IV (PDE IV = hoch affine cAMP-PDE durch cGMP nicht hemmbar, Rolipram-sensitiv) angesehen [H. Hidaka et al., Adv. Cycl. Nucl. Res. 13, 145 (1984); Tips 5, 237 (1984); R. Weishaar et al., J. Med. Chem. 28, 537 (1985); S.A. Harrison et al., Molec. Pharmac. 29, 506 (1986); J. Klein-Tebbe et al., Allergologie 12, 12 (1989); C. Schudt et al., Allergologie 12, 12 (1989)].

Deshalb wurde die PDE-Hemmung der erfindungsgemäßen Verbindungen an einer aus humanen Thrombozyten isolierten PDE III bzw. aus humanen neutrophilen polymorphkernigen Zellen (PMN's) sowie aus der Hundetrachea isolierten PDE IV bestimmt. Die Phosphodiesterasen III und IV werden nach Polson et al., Biochem. Pharmacol. 31, 3403 - 3406 (1982) chromatographisch isoliert.

Die Substanzen werden in DMSO gelöst und weiter verdünnt. Aus einer Reihe von bis hundertfach verdünnten Lösungen werden jeweils 2,1 µl vorgelegt und mit 212 µl Reaktionsgemisch versetzt. Das Reaktionsgemisch enthält Hepes (100 mmol/l), DTE (5 mmol/l), $MgCl_2$ (5 mmol/l), $CaCl_2$ (10 µmol/l), BSA Fraktion V 0,5 mg/ml, cAMP 0,5 µmol/l, 2,8 - $^3$H-cAMP 250000 cpm/ml (0,3 µCi/ml, s.A. 33,5 µCi/mmol), SV (snake venom) 25 µg/212 µl Testansatz).

Durch Zugabe von 10 µl PDE zum Reaktionsgemisch und zur Substanzvorlage wird die Reaktion gestartet. Dann wird für 20 Minuten bei 37°C inkubiert. Die Reaktion wird durch Erhitzen auf 95°C für 45 Sekunden gestoppt. Nach Abkühlen der Proben wird die SV-Lösung zugegeben, die das entstandene 5-AMP spaltet. Nach einer Inkubation von 30 Minuten bei 37°C wird diese Reaktion durch Zugabe von 1 ml Anionenaustauschersuspension gestoppt. Dadurch wird das verbliebene cAMP gebunden. Das entstandene Adenosin bleibt im Überstand nach Zentrifugation und kann in einem Aliquot durch die darin enthaltene Radioaktivität gemessen werden. Die $IC_{50}$-Werte werden im Hill plot mit linearer Regression bestimmt.

In den Tabellen 5, 6 und 7 sind die negativen Logarithmen der gefundenen $IC_{50}$-Werte und die Quotienten aus dem für Theophyllin ermittelten $IC_{50}$-Wert und den $IC_{50}$-Werten der erfindungsgemäßen Substanzen angeführt. Die erfindungsgemäßen Substanzen hemmen die PDE III bzw. PDE IV bedeutend stärker als Theophyllin.

Tabelle 5:

| Hemmung von PDE III | | |
|---|---|---|
| Lfd. Nr. | $-lg[IC_{50}]_{PDE\ III}$ | $[IC_{50}]_{Theophyllin}$ / $[IC_{50}]_{Substanz}$ |
| 2 | 4,98 | 15,5 |
| 3 | 5,28 | 30,8 |
| 4 | 5,22 | 26,9 |
| 6 | 5,20 | 25,7 |
| 7 | 5,05 | 18,2 |
| 9 | 4,91 | 13,2 |
| 10 | 4,97 | 15,1 |
| Theophyllin | 3,79 | 1 |

Tabelle 6:

| Hemmung der PDE IV aus Hundetrachea | | |
|---|---|---|
| Lfd. Nr. | $-lg[IC_{50}]_{PDE\ IV}$ Hundetrachea | $[IC_{50}]_{Theophyllin}$ / $[IC_{50}]_{Substanz}$ |
| 2 | 5,79 | 95,6 |
| 3 | 5,75 | 82,2 |
| 4 | 6,36 | 355 |
| 7 | 5,32 | 32,4 |
| 8 | 5,66 | 70,8 |
| 9 | 5,65 | 69,2 |
| 10 | 5,68 | 74,1 |
| 11 | 5,89 | 120 |
| 12 | 5,80 | 97,8 |
| 15 | 5,70 | 77,7 |
| Theophyllin | 3,81 | 1 |

Tabelle 7:

| Hemmung der PDE IV aus humanen neutrophilen polymorphkernigen Zellen (PMN's) | | |
|---|---|---|
| Lfd. Nr. | $-lg[IC_{50}]_{PDE\ IV}$ PNM's | $[IC_{50}]_{Theophyllin}$ / $[IC_{50}]_{Substanz}$ |
| 3 | 5,82 | 117 |
| 4 | 6,33 | 357 |
| 11 | 6,22 | 288 |
| 13 | 6,18 | 263 |
| 14 | 5,69 | 85,1 |
| 16 | 6,50 | 550 |
| 17 | 6,47 | 513 |
| 18 | 6,64 | 759 |
| 19 | 6,48 | 525 |
| 20 | 5,93 | 148 |
| Theophyllin | 3,76 | 1 |

**Ansprüche**

1. 6-Aryl-3-cyanaminopyradazine der allgemeinen Formel I

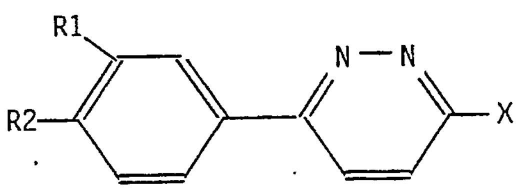

(I),

worin einer der Substituenten R1 und R2 Methoxy, Difluormethoxy oder Ethoxy, und der andere C1-C5-Alkoxy, C4-C7-Cycloalkoxy, C3-C7-Cycloalkylmethoxy, C3-C5-Alkenyloxy oder C1-C4-Polyfluoralkoxy bedeutet, X Cyanamino bedeutet, und ihre Salze mit Basen.

2. Verbindungen der Formel I nach Anspruch 1, worin
R1 Methoxy, Difluormethoxy oder Ethoxy,
R2 C1-C4-Alkoxy, C4-C6-Cycloalkoxy, C3-C6-Cycloalkylmethoxy, C3-C4-Alkenyloxy oder C1-C2-Polyfluoralkoxy und
X Cyanamino bedeuten,
und ihre Salze mit Basen.

3. Verbindungen der Formel I nach Anspruch 1, worin
R1 C2-C4-Alkoxy, C4-C6-Cycloalkoxy, C3-C6-Cycloalkylmethoxy, C3-C4-Alkenyloxy oder C1-C2-Polyfluoralkoxy,
R2 Methoxy, Difluormethoxy oder Ethoxy und
X Cyanamino bedeuten,
und ihre Salze mit Basen.

4. Verbindungen der Formel I nach Anspruch 1, worin einer der Substituenten R1 und R2 Methoxy, Difluormethoxy oder Ethoxy, und der andere C1-C4-Cycloalkoxy, C4-C6-Cycloalkoxy, C3-C6-Cycloalkylmethoxy oder C1-C2-Polyfluoralkoxy bedeutet und X Cyanamino bedeutet, und ihre Salze mit Basen.

5. Verbindungen der Formel I nach Anspruch 1, worin R1 C2-C4-Alkoxy, Cyclopentyloxy, Cyclopropylmethoxy, Cyclobutylmethoxy, Difluormethoxy oder 2,2,2-Trifluorethoxy bedeutet, R2 Methoxy, Ethoxy oder Difluormethoxy bedeutet und X Cyanamino bedeutet, und ihre pharmakologisch verträglichen Salze mit Basen.

6. Verfahren zur Herstellung der 6-Aryl-3-cyanaminopyridazine nach Anspruch 1, dadurch gekennzeichnet, daß man eine Verbindung der allgemeinen Formel I, worin R1 und R2 die in Anspruch 1 angegebenen Bedeutungen haben und X eine nucleophil verdrängbare Abgangsgruppe bedeutet, mit Alkalicyanamid in Gegenwart eines Phasentransferkatalysators in einem wasserfreien, inerten Lösungsmittel umsetzt.

7. Arzneimittel enthaltend eine oder mehrere Verbindungen nach einem der Ansprüche 1, 2, 3, 4 oder 5 und/oder ihre pharmakologisch verträglichen Salze.

8. Verbindungen nach einem der Ansprüche 1, 2, 3, 4 oder 5 und/oder ihre pharmakologisch verträglichen Salze zur Anwendung bei der Behandlung und/oder Prophylaxe von Erkrankungen der Bronchien.

9. Verwendung der Verbindungen nach einem der Ansprüche 1, 2, 3, 4 oder 5 und/oder ihrer pharmakologisch verträglichen Salze zur Herstellung von Arzneimitteln zur Behandlung und/oder Prophylaxe von Erkrankungen der Bronchien.

Europäisches Patentamt

# EUROPÄISCHER RECHERCHENBERICHT

Nummer der Anmeldung

EP 90 10 6990

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | JOURNAL OF MEDICINAL CHEMISTRY, Band 25, Nr. 7, Juli 1982, Seiten 821-824, The American Chemical Society; C.B. CHAPLEO et al.: "Synthesis of some potential antihypertensive phthalazinyl- and quinoxalinylguanidines" * Verbindungen 2,9 * --- | 1 | C 07 D 237/00<br>A 61 K 31/50 |
| A | J. HETEROCYCLIC CHEM., Band 19, Mai-Juni 1982, Seiten 577-583; B. STANOVNIK et al.: "Reactions of N-heteroarylformamide oximes and N-heteroarylacetamide oximes with N,N-dimethylformamide dimethyl acetal. Synthesis of 2-methyl-s-triazolo[1,5,-chi]azines and N-methylcyanoaminoazines" * Seite 579, Verbindung 4b * ----- | 1 | |
| | | | **RECHERCHIERTE SACHGEBIETE (Int. Cl.5)** |
| | | | C 07 D 237/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 10-07-1990 | DE JONG B.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)